# FASCICULE DE BREVET EUROPEEN

(11) **EP 1 588 691 B1**
(45) Date de publication et mention de la délivrance du brevet: **09.01.2008**
(21) Numéro de dépôt: 05300301.8
(22) Date de dépôt: 20.04.2005
(51) Int. Cl.: A61K 8/46, A61K 8/58, A61Q 5/04

(54) **Composition pour la déformation permanente des cheveux contenant un réducteur et un photooxydant**
Zusammensetzung zur dauerhaften Haarverformung enthaltend ein reduzierendes Mittel und ein photo-oxidierendes Mittel
Permanent hair shaping composition containing a reducing agent and a photo-oxidant

(30) Priorité: 22.04.2004 FR 0450764
(43) Date de publication de la demande: 26.10.2005
(73) Titulaire: L'ORÉAL, 75008 Paris (FR)
(72) Inventeur: Livoreil, Aude, 75006, PARIS (FR); Vic, Gabin, 60280, VENETTE (FR); Samain, Henri, 91570, BIEVRES (FR)
(74) Mandataire: Catherine, Alain

(56) Documents cités:
- EP-A- 0 673 643
- WO-A-00/52101
- WO-A-99/13823
- CH-A- 307 483
- DE-A- 19 905 498
- US-A- 2 742 909
- US-A- 5 032 291
- DATABASE CAPLUS [Online] CHEMICAL ABSTRACTS SERVICE, COLUMBUS, OHIO, US; XP002306702 extrait de STN Database accession no. 2003:877114
- DATABASE CAPLUS [Online] CHEMICAL ABSTRACTS SERVICE, COLUMBUS, OHIO, US; XP002306703 extrait de STN Database accession no. 1990:597605
- DATABASE WPI Section Ch, Week 198830 Derwent Publications Ltd., London, GB; Class D21, AN 1988-209062 XP002306704 & JP 63 145213 A (KANEBO LTD) 17 juin 1988 (1988-06-17)

## Description

La présente invention se rapporte à une composition cosmétique réductrice, pour le premier temps d'une opération de déformation permanente des cheveux comprenant au moins un agent réducteur, et au moins un photooxydant. Elle a également pour objet un procédé de déformation permanente des cheveux mettant en oeuvre ladite composition.

La technique la plus usuelle pour obtenir une déformation permanente des cheveux consiste, dans un premier temps à réaliser l'ouverture des liaisons disulfures de la kératine (cystine) à l'aide d'une composition contenant un agent réducteur, puis après avoir de préférence rincé les cheveux, à reconstituer dans un second temps les dites liaisons disulfures en appliquant sur les cheveux préalablement lissés ou mis sous tension par des moyens adéquats tels que des bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Ces technologies courantes de transformation de la forme de cheveux, notamment les compositions de permanente ou de lissage des cheveux, à base de produits thiolés, conduisent à des performances de mise en forme satisfaisantes, mais confèrent une odeur désagréable aux cheveux.

Pour remédier à cet inconvénient, une première solution, mise en oeuvre dans les compositions classiques, consiste à masquer les odeurs par addition de parfum. Cette solution n'est cependant pas satisfaisante car elle ne supprime pas les mauvaises odeurs.

Des solutions alternatives ont été proposées.

Ainsi, des systèmes de piégeage physique d'odeur ont été proposés, notamment par des zéolites. Par exemple, la demande de brevet US 5,184,630 propose d'ajouter une zéolite à une composition cosmétique, pour réduire les mauvaises odeurs.

Mais la réduction d'odeur par ces systèmes de piégeage physique est insuffisante.

Par ailleurs, il a été proposé de remplacer l'eau oxygénée utilisée dans l'étape de fixation des procédés de déformation permanente des cheveux par une catalyse au manganèse.

Mais lorsque l'on réalise successivement une opération de mise en forme permanente des cheveux suivie d'une coloration des cheveux, ces compositions induisent une perturbation de la montée de couleur : la coloration obtenue est trop intense.

Il existe donc un besoin pour de nouvelles compositions cosmétiques destinées à une opération de déformation permanente des cheveux possédant une grande capacité de diminution des mauvaise odeurs, et qui ne présentent pas les inconvénients de l'art antérieur, notamment qui n'induisent pas de perturbation de la montée de couleur lors d'opérations successives de mise en forme permanente des cheveux et de coloration des cheveux.

La Demanderesse a découvert de façon surprenante que l'utilisation dans une composition cosmétique pour la déformation permanente des fibres kératiniques d'un photooxydant complexé avec au moins un métal conduit à une diminution importante des mauvaises odeurs sans pour autant induire une perturbation de la montée de la couleur dans la cas d'une succession permanente/coloration, et permet d'obtenir une frisure satisfaisante en intensité.

Les photooxydants catalysent la décomposition des molécules de thiols de faible taille par l'oxygène de l'air, et réduisent efficacement l'odeur désagréable.

En effet, les photooxydants sont des molécules ou des complexes susceptibles de déclencher une réaction d'oxydation après illumination, le plus souvent dans le domaine de la lumière visible entre 400 nm et 800 nm.

L'exposition à la lumière d'un photooxydant le fait passer dans un état d'énergie élevée. Le photooxydant ainsi excité transfère alors son énergie aux molécules d'oxygène présentes dans le milieu, ce qui les transforme en oxygène activé. Cette forme d'oxygène est très réactive et peut oxyder facilement d'autres espèces chimiques présentes dans le milieu, en particulier certaines espèces soufrées.

L'invention a donc d'abord pour objet une composition cosmétique réductrice pour la déformation permanente des fibres kératiniques, en particulier des cheveux, comprenant, dans un milieu cosmétiquement acceptable, au moins un agent réducteur et au moins un photooxydant complexé avec au moins un métal.

Le métal est de préférence choisi parmi les métaux de transition.

En particulier, les métaux de transition peuvent être Ti, V, Cr, Mn, Fe, Co, Ni, Cu, Zn, Mo, Ru, Rh, Pd et Ag, de préférence Cu, Zn, Ni, Fe, Co et Mn, mieux Fe et Mn.

Le métal peut encore être choisi parmi Si, Al, Ga, Ge et Sn.

Le métal complexant le photooxydant est généralement apporté sous forme de sel métallique, d'oxyde métallique ou de complexe métallique avec un ou plusieurs ligands.

Ainsi, selon un premier mode de réalisation de l'invention, le métal est apporté sous la forme d'un sel métallique de formule : M(T)ₙ (I)où
M est un métal choisi parmi Ti, V, Cr, Mn, Fe, Co, Ni, Cu, Zn, Mo, Ru, Rh, Pd et Ag,
T est un anion inorganique choisi parmi les ions sulfate, sulfonate, phosphate, phosphonate, chlorure, bromure et nitrate, ou un anion organique choisi parmi les ions lactate, citrate, acétate, et
n est un entier variant de 1 à 8, de préférence égal à 2 ou 3.

Selon un deuxième mode de réalisation de l'invention, le métal est apporté sous la forme d'un oxyde métallique de formule :

M_{m'}(O)ₘ (II)

où
M est un métal choisi parmi Ti, V, Cr, Mn, Fe, Co, Ni, Cu, Zn, Mo, Ru, Rh, Pd et Ag,
m' est entier variant de 1 à 5, et m est un entier variant de 1 à 8, de préférence égal à 2 ou 3.

Selon un troisième mode de réalisation de l'invention, le métal est apporté sous la forme d'un complexe métallique avec un ou plusieurs ligands de formule :

M(L)ₚ (III)

où
M est un métal choisi parmi Ti, V, Cr, Mn, Fe, Co, Ni, Cu, Zn, Mo, Ru, Rh, Pd et Ag,
L est un ligand organique choisi parmi les composés cycliques ou acycliques, saturés ou insaturés, comprenant des groupes carboxylate, et/ou amines primaires, secondaires ou tertiaires, et/ou hydroxy, ou un ligand inorganique choisi parmi NH₃ et NO, et
p est un entier variant de 1 à 10, de préférence égal à 2 ou 3.

De préférence, L est un ligand organique choisi parmi la pyridine, la pyridazine, la pyrimidine, la pyrazine, l'imidazole, le pyrazole, le triazole, la 2,2'-bispyridylamine, la tris-(2-pyridylméthyl)amine, le 1,4,7-triazaclyclononane, le 1,4,7-triméthyl-1,4,7-triazaclyclononane, le 1,5,9-triméthyl-1,5,9-triazacyclododécane, la ((bis-(1-méthylimidazol-2-yl)-méthyl))-(2-pyridylméthyl)-amine, la N,N'-((bis-(1-méthylimidazol-2-yl)-méthyl))-éthylènediamine, le N-bis-(2-benzimidazolylméthyl)-aminoéthanol, le 2,6-bis-(bis-(2-benzimidazolylméthyl)aminométhyl)-4-méthylphénol, le N,N,N',N'-tetrakis-(2-benzimidazolylméthyl)-2-hydroxy-1,3-diaminopropane, le 2,6-bis(bis-(2-pyridylméthyl)aminométhyl)-4-méthylphénol, le 1,3-bis(bis-(2-benzimidazolylméthyl)aminométhyl)-benzol, le sorbitol, le mannitol, l'érythritol, l'adonitol, l'inositol, le lactose, et éventuellement leurs sels.

De préférence encore, le ligand organique est choisi parmi le 1,4,8 ; 11-tétraazacyclotétradécane, le 1,8-diazabicydo[5.4.0]undec-7-ène, l'éthylènediamine, l'acide nitrilotriacétique, la tris(aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylméthyl)amine, la tris(2-pyridyléthyl)amine, et la N-carboxyméthyl-N-(2-pyridylméthyl)glycine.

Il est toutefois préférable d'utiliser directement dans la composition selon l'invention un photooxydant préalablement complexé.

Comme expliqué précédemment, la composition selon l'invention comprend au moins un photooxydant complexé avec au moins un métal. Dans le photooxydant complexé, ledit métal est à un degré d'oxydation supérieur à 0.

Avantageusement, le ou les photooxydants sont choisis parmi :
- les dérivés de porphyrines choisis parmi les chlorines, les phéophorbides, les pyréophorbides et les benzoporphyrines,
- les naphtalocyanines,
- les phtalocyanines, et
- la cyanocobalamine.

Selon un mode de réalisation préféré, la ou les phtalocyanines complexées avec au moins un métal sont choisies parmi les composés de formule : 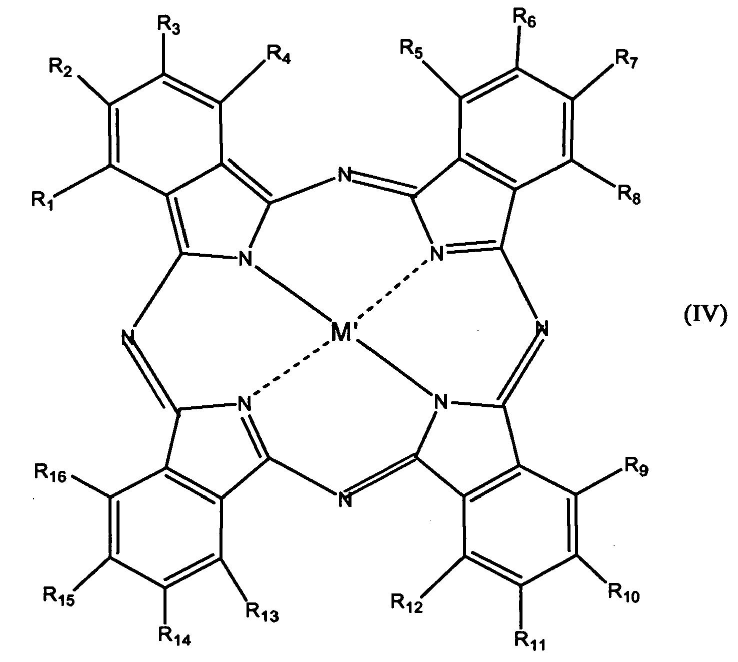 où
M' est un métal choisi parmi Ti, V, Cr, Mn, Fe, Co, Ni, Cu, Zn, Mo, Ru, Rh, Pd et Ag, de préférence Cu, Zn, Ni, Fe, Co et Mn, mieux Fe et Mn, ledit métal M' étant à un degré d'oxydation supérieur à 0, et étant éventuellement accompagné d'un ou plusieurs anions organiques ou inorganiques tels que définis précédemment et/ou d'un ou plusieurs ligands tels que définis précédemment,
R₁ à R₁₆ représentent indépendamment :
- soit l'hydrogène,
- soit des groupements hydroxy, amino, mono ou dialkylamino, mono ou dihydroxyalkylamino, alcoxy, halogène, cyano,
- soit des chaînes hydrocarbonées comportant de 1 à 50 atomes de carbones, saturées ou insaturées, linéaires ou ramifiées, éventuellement interrompues par un ou plusieurs hétéroatomes tels que S, O, N, P, par un ou plusieurs cycles aromatiques ou hétéroaromatiques, éventuellement substitués, notamment par des groupes hydroxy ou alkylhydroxy, ou par un plusieurs groupements carbonyle, lesdites chaînes étant éventuellement substituées par un ou plusieurs groupements choisis parmi les groupements hydroxy, amino, alcoxy, halogène, cyano, mono ou dialkylamino, mono ou dihydroxyalkylamino,
- soit des radicaux portant des groupes ioniques solubilisants,
- soit des radicaux portant un ou plusieurs groupes réactifs aptes à greffer la phtalocyanine sur un support solide ou sur un polymère soluble ou insoluble.

Généralement, les groupes ioniques solubilisants sont choisis parmi les groupes carboxylate, sulfonate, trialkylammonium, trialkylphosphonium, triarylphosphonium, alkylpyridinium et arylpyridinium, les restes alkyles étant en C₁-C₁₈ et les restes aryles en C₆-C₁₂.

Comme phtalocyanines utilisables selon l'invention, on peut citer en particulier la phtalocyanine de cuivre II commercialisée par la société Aldrich sous la référence 61218, la phtalocyanine de manganèse II commercialisée par la société Aldrich sous la référence 37,955-7, et la phtalocyanine de Fer III commercialisée par la société Fluka sous la référence 44968.

Le ou les photooxydants peuvent être solubles dans la composition ou être en dispersion dans la composition.

Selon un mode de réalisation particulièrement préféré, le ou les photooxydants sont immobilisés sur un support solide. Cela permet de limiter leur diffusion dans les fibres kératiniques.

Ainsi, le ou les photooxydants peuvent être adsorbés sur un support solide, ou greffés, généralement par liaison covalente, sur ce support.

Généralement, le support solide est choisi parmi les particules de silice, d'oxyde de titane, de polymère et de métaux.

Parmi les particules de polymères pouvant être utilisées comme support, on peut citer les particules de polypropylène, de polyuréthane, ou de styrène-divinylbenzène.

Des procédés de greffage ou d'adsorption de phtalocyanines sur différents supports sont notamment décrits dans les publications suivantes :
- « Synthesis and catalytic properties of a novel phthalocyanine covalently grafted onto silica" S. Mangematin, AB Sorokin, Journal of Porphyrins and Phthalocyanines, 2001, 5, p 674-680 ;
- "Fabrication of deodorizing fabric by grafting metal phthalocyanine derivative onto non woven polypropylene fabric", Journal of Applied Polymer Science, 2001, 82(4), p839-846;
- "Fabrication of gas sensing films based on nanoparticles grafted with electroncially or chemically interacting organic molecules" WO 2000014520, CEA
- "in vivo comparison of blood compatiblity of iron-phthalocyanine grafted polyurethanes", Advances in Biomaterials, 1980, 1, p519-527;
- "Macroporous styrene-divinylbenzene copolymers as carriers for polyvinylamine-cobalt phthalocyanine oxidation catalysts", Angevirandte Makromolekulare Chemie, 1980, 89, p201-219.

L'immobilisation du ou des photooxydants peut également se faire par piégeage.

Ainsi, le ou les photooxydants peuvent être emprisonnés dans un matériau poreux choisis de préférence parmi les particules de polymère, tels que les polyamides du type ORGASOL, les réseaux tridimensionnels obtenus par condensation de molécules d'alcoxysilanes et les zéolites.

Les réseaux tridimensionnels utilisables sont par exemple ceux décrits dans la publication suivante : "Encapsulation of iron phthalocyanine in sol-gel materials" AB Sorokin, P. Buisson, AC pierre, Microporous and Mesoporous materials, 2001, 46, p87-98.

Généralement, le ou les photooxydants représentent 0,1 à 20%, de préférence 1 à 10%, en poids du poids total de la composition.

Comme expliqué précédemment, la composition selon l'invention comprend, outre un ou plusieurs photooxydants complexés avec au moins un atome de métal, un ou plusieurs agents réducteurs.

Généralement, le ou les agents réducteurs sont choisis parmi les agents réducteurs de formule :

(H)_{q} X (R)ᵣ (VI)

dans laquelle X représente P, S ou SO₂, q vaut 0 ou 1, r vaut 1 ou 2 ou 3 et R est un radical (C₁-C₂₀)hydrocarboné, linéaire ou ramifié, saturé ou insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement ((C₁-C₃₀)acyle) amino, un groupement mono ou di((C₁-C₃₀)alkyl)amino, un groupement mono ou dihydroxy ((C₁-C₃₀)alkyl)amino.
ou l'un de ses sels en combinaison avec une base.

En particulier, on peut citer l'acide thioglycolique ou l'acide thiolactique et leurs dérivés esters et amides, notamment le monothioglycolate de glycérol, la cystéamine et ses dérivés (C₁-C₄) acylés tels que la N-acétyl-cystéamine ou la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides tels que ceux décrits dans la demande de brevet EP-A-354 835, les N-mono ou N,N-dialkylmercapto-4-butyramides tels que ceux décrits dans la demande de brevet EP-A-368 763, les aminomercaptoalkylamides, tels que ceux décrits dans la demande de brevet EP-A-432 000, les dérivés des acides N-(mercaptoalkyl)succinamiques et des N-(mercaptoalkyl)succinimides tels que ceux décrits dans la demande de brevet EP-A-465 342, les alkylamino mercaptoalkylamides tels que ceux décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides tels que ceux décrits dans la demande de brevet FR-A-2 692 481, les N-mercaptoalkylalcanediamides décrits dans la demande de brevet EP-A-653 202, ainsi que les dérivés d'acide formamidine sulfinique. Le cas échéant, ces composés peuvent être utilisés sous forme de sels.

Généralement, le ou les agents réducteurs représentent 0,05 à 30%, de préférence de 1 à 20%, en poids du poids total de la composition.

Le pH de la composition selon l'invention est de préférence compris entre 4 et 11, et plus particulièrement entre 6 et 10.

Le pH est généralement obtenu à l'aide d'un agent alcalin tel que, par exemple, l'ammoniaque, la monoéthanolamine, la diéthanolamine, la triéthanolamine, la propanediamine-1,3, un carbonate ou bicarbonate alcalin ou d'ammonium, un carbonate organique tel que le carbonate de guanidine, un hydroxyde alcalin, ou à l'aide d'un agent acidifiant tel que par exemple l'acide chlorhydrique, l'acide acétique, l'acide lactique, l'acide oxalique ou l'acide borique, ou bien encore à l'aide de tampons usuels, tels que par exemple les tampons borate, phosphate ou TRIS (à base de tris(hydroxyméthyl) amino méthane).

Dans un mode de réalisation préféré, la composition réductrice selon l'invention contient également au moins un agent tensioactif de type non-ionique, anionique, cationique ou amphotère. Parmi ceux-ci, on peut citer les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés ainsi que d'autres tensioactifs non-ioniques du type hydroxypropyléthers.

Lorsque la composition réductrice selon l'invention contient un ou plusieurs agents tensioactifs, celui-ci ou ceux-ci représentent généralement au plus 30 % en poids, et de préférence entre 0,5 et 10 % en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les brevets français FR2598613 et FR 2 470 596. On peut également utiliser comme agents traitants des silicones volatiles ou non, linéaires ou cycliques, et leurs mélanges, les polydiméthylsiloxanes, les polyorganosiloxanes quaternisés tels que ceux décrits dans la demande de brevet français FR2535730, les polyorganosiloxanes à groupements aminoalkyles modifiés par des groupements alcoxycarbonylalkyles tels que ceux décrits dans le brevet US 4,749,732, des polyorganosiloxanes tels que le copolymère polydiméthylsiloxane-polyoxyalkyle du type diméthicone copolyol, un polydiméthylsiloxane à groupements terminaux stéaroxy (stéaroxy-diméthicone), un copolymère polydiméthylsiloxane dialkylammonium acétate ou un copolymère polydiméthyl-siloxane polyalkylbétaïne décrits dans le brevet britannique n° 2 197 352, des polysiloxanes organo modifiés par des groupements mercapto ou mercaptoalkyles tels que ceux décrits dans le brevet français FR 1 530 369 et dans la demande de brevet européen EP 295 780, ainsi que des silanes tels que le stéaroxytriméthylsilane.

La composition réductrice selon l'invention peut également contenir un ou plusieurs autres ingrédients traitants tels que des polymères cationiques tels que ceux utilisés dans les compositions des brevets français n° 79.32078 (FR 2 472 382) et n° 80.26421 (FR 2 495 931), et en particulier des polymères cationiques du type ionène ou des cyclopolymères, des aminoacides basiques tels que la lysine ou l'arginine, des aminoacides, acides tels que l'acide glutamique ou l'acide aspartique, des peptides et leurs dérivés, des hydrolysats de protéines ou des cires.

La composition réductrice selon l'invention peut également contenir des agents de gonflement et de pénétration, ou des agents permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition selon l'invention comprend généralement un ou plusieurs solvants choisis parmi l'eau, les alcools en C₁-C₆, de préférence les alcanols tels que l'éthanol, le propanol et l'isopropanol, les alcanediols tels que l'éthylèneglycol, le propylène glycol et le pentanediol, l'alcool benzylique, les éthers en C₂-C₆, les esters en C₂-C₆, la N-méthylpyrrolidone (NMP), et les cétones en C₃-C₆.

De préférence, la composition se présente sous forme aqueuse, de préférence sous la forme d'une lotion, épaissie ou non, d'une crème, épaissie ou non, ou d'un gel.

L'invention a encore pour objet un procédé de déformation permanente des cheveux comprenant :
- une étape d'application sur les cheveux d'une composition réductrice telle que définie précédemment, pour réduire les liaisons disulfures de la kératine, puis
- une étape de fixation par oxydation, pour reformer lesdites liaisons, par application d'une composition oxydante, pour reformer lesdites liaisons, par application d'une composition oxydante sur les cheveux ou par mise en contact des cheveux avec l'oxygène de l'air.

De préférence, le ou les photooxydants sont incorporés dans la composition réductrice au moment de l'emploi de la composition.

Selon un autre mode de réalisation, le ou les photooxydants peuvent être appliqués sur les cheveux avant l'application du ou des agents réducteurs.

Ainsi, l'invention a encore pour objet un procédé de déformation permanente des cheveux comprenant :
- une étape d'application sur les cheveux d'une composition cosmétique comprenant, dans un milieu cosmétiquement acceptable, au moins un photooxydant complexé avec au moins un métal, puis
- une étape d'application sur les cheveux d'une composition réductrice comprenant, dans un milieu cosmétiquement acceptable, au moins un agent réducteur,
- une étape de fixation par oxydation, pour reformer lesdites liaisons, par application d'une composition oxydante sur les cheveux ou par mise en contact des cheveux avec l'oxygène de l'air.

La photooxydation peut être déclenchée par exposition des cheveux à la lumière naturelle ou artificielle.

Les cheveux sont mis en forme en utilisant des moyens mécaniques bien connus de l'homme de l'art.

Lorsque l'on souhaite réaliser une permanente, on utilise de préférence des bigoudis, la composition réductrice étant appliquée avant ou après les moyens de mise en forme des cheveux, et la composition oxydante de fixation étant appliquée après la composition réductrice, avec ou sans étape intermédiaire ou subséquente de rinçage ou d'application de composition intermédiaire.

De préférence, on applique une composition réductrice selon l'invention sur des cheveux mouillés préalablement enroulés sur des rouleaux ayant de 2 à 30 mm de diamètre. La composition peut également être appliquée au fur et à mesure de l'enroulage des cheveux. Généralement, on laisse ensuite agir la composition réductrice pendant un temps de 5 à 60 minutes, de préférence de 15 à 45 minutes, puis on rince abondamment. On applique alors, sur les cheveux enroulés, la composition oxydante permettant de reformer les liaisons disulfures de la kératine, pendant un temps de pose de 2 à 10 minutes. Après avoir enlevé les rouleaux, on rince abondamment la chevelure.

On peut également, après application de la composition réductrice, soumettre la chevelure à un traitement thermique par chauffage à une température comprise entre 30 et 60°C pendant tout ou partie du temps de pose. Dans la pratique, cette opération peut être conduite au moyen d'un casque de coiffure, d'un sèche cheveux, d'un générateur de rayons infrarouges et d'autres appareils chauffants classiques.

On peut notamment utiliser, à la fois comme moyen de chauffage et de mise en forme de la chevelure, un fer chauffant à une température comprise entre 60 et 250°C et de préférence entre 120 et 220°C, l'utilisation du fer chauffant se faisant entre l'étape d'application de la composition réductrice et l'étape de fixation.

Lorsque l'on souhaite effectuer le défrisage ou le décrêpage des cheveux, on applique sur les cheveux une composition réductrice selon l'invention, puis l'on soumet les cheveux à une déformation mécanique permettant de les fixer dans leur nouvelle forme, par une opération de lissage des cheveux avec un peigne à larges dents, avec le dos d'un peigne ou à la main. Après généralement un temps de pose de 5 à 60 minutes, de préférence de 15 à 45 minutes, on procède à un nouveau lissage, puis on rince soigneusement et on applique la composition oxydante telle que définie ci-dessus, qu'on laisse agir pendant environ 2 à 10 minutes, puis on rince abondamment les cheveux.

La composition oxydante peut être toute composition oxydante couramment utilisée pour la déformation permanente des cheveux et contient un agent oxydant choisi de préférence parmi l'eau oxygénée, les bromates alcalins, les persels, les polythionates et un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 %, en poids par rapport au poids total de la composition oxydante.

Le pH de la composition oxydante est généralement compris entre 2 et 10.

Comme expliqué précédemment, l'application de la composition oxydante peut être effectuée immédiatement ou être différée.

Selon un mode de réalisation particulier des procédés selon l'invention, le lissage des cheveux peut également être effectué, en tout ou partie, à l'aide d'un fer chauffant entre 60 et 250°C, et de préférence entre 120 et 220°C.

L'invention concerne enfin un kit pour la déformation permanente des cheveux comprenant dans un premier compartiment une composition réductrice telle que définie précédemment, et dans un second compartiment une composition oxydante.

La présente invention est illustrée par les exemples suivants.

### Exemples

Les composés photooxydants suivants sont utilisés pour formuler des compositions réductrices selon l'invention :
- Phtalocyanine de Manganèse II (notée P(Mn))
- Phtalocyanine de Fer III (notée P(Fe))
On formule les compositions réductrices suivantes :

| Composition réductrice A1 (ne contenant pas de photooxydant) | |
|---|---|
| Acide thioglycolique: | 9 g |
| NH₄OH: | qsp pH 9 |
| Eau: | qsp 100 g |

| Composition réductrice A2 (selon l'invention) | |
|---|---|
| Acide thioglycolique: | 9 g |
| P(Mn): | 5 g |
| NH₄OH: | qsp pH 9 |
| Eau: | qsp 100 g |

| Composition réductrice A3 (selon l'invention) | |
|---|---|
| Acide thioglycolique: | 9 g |
| P(Fe): | 5 g |
| NH₄OH: | qsp pH 9 |
| Eau: | qsp 100 g |

| Composition de fixation B | |
|---|---|
| H₂O₂: | 8 vol |
| Acide citrique: | qsp pH 3 |
| Eau: | qsp 100 g |

On réalise la mise en forme de cheveux au moyen des compositions réductrices précédentes et de la composition de fixation.

Pour cela, les cheveux sont lavés et essorés, et mis sous tension au moyen de bigoudis.

On applique la composition réductrice A1 ou A2 ou A3, suivie d'une pause de 15 minutes à température ambiante (environ 20°C), sous un film plastique transparent qui permet de contenir l'odeur dégagée par l'application de la composition réductrice.

On retire le film plastique et l'odeur est évaluée.

Puis les cheveux sont rincés à l'eau.

On applique ensuite sur les cheveux la composition B de fixation. On laisse poser pendant 5 minutes à température ambiante.

Les cheveux sont alors rincés, les bigoudis défaits et la frisure est évaluée.

On constate que pour les cheveux traités avec la composition A1, l'odeur désagréable est beaucoup plus forte que pour les cheveux traités avec la composition A2, ou A3, pour une frisure équivalente après fixation par B.

## Revendications

1. Composition cosmétique réductrice pour la déformation permanente des fibres kératiniques, en particulier des cheveux, **caractérisée en ce qu'**elle comprend, dans un milieu cosmétiquement acceptable, au moins un agent réducteur et au moins un photooxydant complexé avec au moins un métal de transition choisis parmi Ti, V, Cr, Mn, Fe, Co, Ni, Cu, Zn, Mo, Ru, Rh, Pd et Ag, le ou les photooxydants étant choisis parmi :
- les dérivés de porphyrines choisis parmi les chlorines, les phéophorbides, les pyréophorbides et les benzoporphyrines,
- les naphtalocyanines,
- les phtalocyanines, et
- la cyanocobalamine,
le ou les photooxydants représentant 1 à 10%, en poids du poids total de la composition.

2. Composition selon la revendication 1 **caractérisée en ce que** le métal de transition est choisi parmi Cu, Zn, Ni, Fe, Co et Mn.

3. Composition selon l'une quelconque des revendications précédentes **caractérisée en ce que** le métal est apporté sous forme de sel métallique, d'oxyde métallique ou de complexe métallique avec un ou plusieurs ligands.

4. Composition selon la revendication précédente **caractérisée en ce que** le métal est apporté sous la forme d'un sel métallique de formule :
M(T)ₙ (I)
où
M est un métal choisi parmi Ti, V, Cr, Mn, Fe, Co, Ni, Cu, Zn, Mo, Ru, Rh, Pd et Ag,
T est un anion inorganique choisi parmi les ions sulfate, sulfonate, phosphate, phosphonate, chlorure, bromure et nitrate, ou un anion organique choisi parmi les ions lactate, citrate, acétate, et
n est un entier variant de 1 à 8, de préférence égal à 2 ou 3.

5. Composition selon la revendication 3 **caractérisée en ce que** le métal est apporté sous la forme d'un oxyde métallique de formule:
M_{m'}(O)ₘ (II)
où
M est un métal choisi parmi Ti, V, Cr, Mn, Fe, Co, Ni, Cu, Zn, Mo, Ru, Rh, Pd et Ag,
m' est entier variant de 1 à 5, et m est un entier variant de 1 à 8, de préférence égal à 2 ou 3.

6. Composition selon la revendication 3 **caractérisée en ce que** le métal est apporté sous la forme d'un complexe métallique avec un ou plusieurs ligands de formule :
M(L)ₚ (III)
où
M est un métal choisi parmi Ti, V, Cr, Mn, Fe, Co, Ni, Cu, Zn, Mo, Ru, Rh, Pd et Ag ,
L est un ligand organique choisi parmi les composés cycliques ou acycliques, saturés ou insaturés, comprenant des groupes carboxylate, et/ou amines primaires, secondaires ou tertiaires, et/ou hydroxy, ou un ligand inorganique choisi parmi NH₃ et NO, et
p est un entier variant de 1 à 10, de préférence égal à 2 ou 3.

7. Composition selon la revendication 6 **caractérisée en ce que** le ou les ligands sont choisis parmi le 1,4,8 ; 11-tétraazacyclotétradécane, le 1,8-diazabicyclo[5.4.0]undec-7-ène, l'éthylènediamine, l'acide nitrilotriacétique, la tris(aminoéthyl)amine, le tris(aminométhyl)méthane, le 1,3,5-triaminocyclohexane, la pyridine-2,6-dicarboxylique, l'acide pyridine-2,6-dicarboxylique, la 2,2',6',2"-terpyridine, la 1,10-phénanthroline, la tris(2-pyridylméthyl)amine, la tris(2-pyridyléthyl)amine, et la N-carboxyméthyl-N-(2-pyridylméthyl)glycine.

8. Composition cosmétique selon l'une quelconque des revendications précédentes **caractérisée en ce que** la ou les phtalocyanines complexées avec au moins un métal sont choisies parmi les composés de formule : 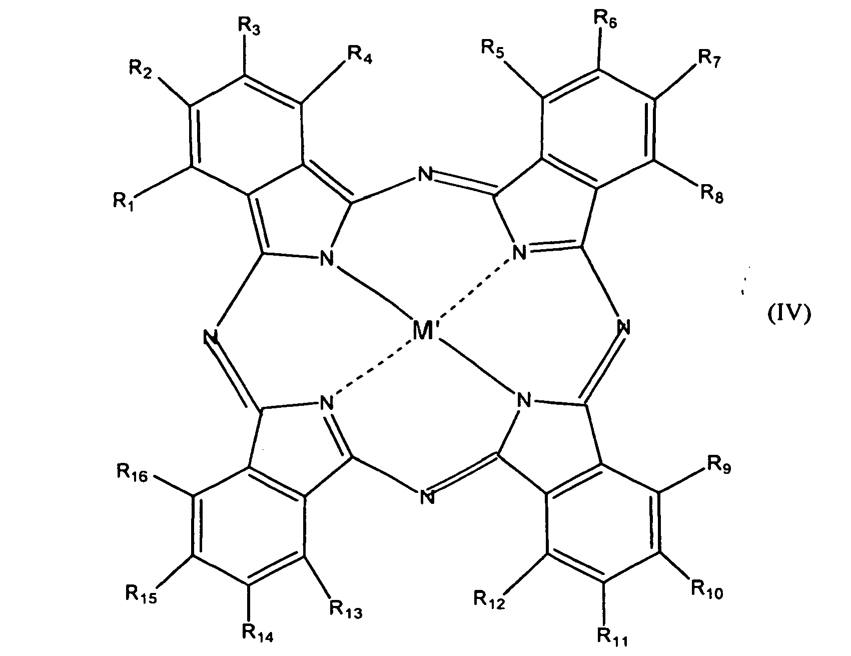 où
M' est un métal choisi parmi les Ti, V, Cr, Mn, Fe, Co, Ni, Cu, Zn, Mo, Ru, Rh, Pd et Ag, de préférence Cu, Zn, Ni, Fe, Co et Mn, mieux Fe et Mn, ledit métal M' étant à un degré d'oxydation supérieur à 0, et étant éventuellement accompagné d'un ou plusieurs anions organiques ou inorganiques tels que définis à la revendication 5 et/ou d'un ou plusieurs ligands tels que définis à la revendication 7,
R₁ à R₁₆ représentent indépendamment :
- soit l'hydrogène,
- soit des groupements hydroxy, amino, mono ou dialkylamino, mono ou dihydroxyalkylamino, alcoxy, halogène, cyano,
- soit des chaînes hydrocarbonées comportant de 1 à 50 atomes de carbones, saturées ou insaturées, linéaires ou ramifiées, éventuellement interrompues par un ou plusieurs hétéroatomes tels que S, O, N, P, par un ou plusieurs cycles aromatiques ou hétéroaromatiques, éventuellement substitués, notamment par des groupes hydroxy ou alkylhydroxy, ou par un plusieurs groupements carbonyle, lesdites chaînes étant éventuellement substituées par un ou plusieurs groupements choisis parmi les groupements hydroxy, amino, alcoxy, halogène, cyano, mono ou dialkylamino, mono ou dihydroxyalkylamino,
- soit des radicaux portant des groupes ioniques solubilisants,
- soit des radicaux portant un ou plusieurs groupes réactifs aptes à greffer la phtalocyanine sur un support solide ou sur un polymère soluble ou insoluble.

9. Composition selon la revendication 8 **caractérisée en ce que** les groupes ioniques solubilisants sont choisis parmi les groupes carboxylate, sulfonate, trialkylammonium, trialkylphosphonium, triarylphosphonium, alkylpyridinium et arylpyridinium, les restes alkyles étant en C₁-C₁₈ et les restes aryles en C₆-C₁₂.

10. Composition selon l'une quelconque des revendications précédentes **caractérisée en ce que** le ou les photooxydants sont solubilisés dans la composition ou sont en dispersion dans la composition.

11. Composition selon l'une quelconque des revendications 1 à 10 **caractérisée en ce que** le ou les photooxydants sont adsorbés ou greffés sur un support solide.

12. Composition selon la revendication 11 **caractérisée en ce que** le support solide est choisi parmi les particules de silice, d'oxyde de titane, de polymère et de métaux.

13. Composition selon la revendication 12 **caractérisée en ce que** les particules de polymères sont choisies parmi les particules de polypropylène, de polyuréthane, ou de styrène-divinylbenzène.

14. Composition selon l'une quelconque des revendications 1 à 10 **caractérisée en ce que** le ou les photooxydants sont emprisonnés dans un matériau poreux choisis de préférence parmi les particules de polymère, les réseaux tridimensionnels obtenus par condensation de molécules d'alcoxysilanes et les zéolites.

15. Composition selon l'une quelconque des revendications précédentes **caractérisée en ce que** le ou les agents réducteurs sont choisis parmi les agents réducteurs de formule :
(H)_{q} X (R)ᵣ (VI)
dans laquelle X représente P, S ou SO₂, q vaut 0 ou 1, r vaut 1 ou 2 ou 3 et R est un radical (C₁-C₂₀)hydrocarboné, linéaire ou ramifié, saturé ou insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement ((C₁-C₃₀)acyle) amino, un groupement mono ou di((C₁-C₃₀) alcoxy)carbonyle, un groupement mono ou dihydroxy ((C₁-C₃₀)alkyl)amino.
ou l'un de ses sels en combinaison avec une base.

16. Composition selon l'une quelconque des revendications précédentes **caractérisée en ce que** le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

17. Composition selon l'une quelconque des revendications précédentes **caractérisée en ce que** le ou les agents réducteurs représentent de 0,05 à 30%, de préférence de 1 à 20%, en poids du poids total de la composition.

18. Composition selon l'une quelconque des revendications précédentes, **caractérisée en ce qu'**elle comprend au moins un agent tensioactif choisi parmi les alkyl sulfates, les alkyl benzènesulfates, les alkyl éthersulfates, les alkyl sulfonates, les sels d'ammonium quaternaire, les alkyl bétaïnes, les alkyl phénols oxyéthylénés, les alcanolamides d'acides gras, les esters d'acides gras oxyéthylénés, les hydroxypropyléthers.

19. Composition selon l'une quelconque des revendications précédentes **caractérisée en ce qu'**elle comprend un ou plusieurs solvants choisis parmi l'eau, les alcools en C₁-C₆, de préférence les alcanols tels que l'éthanol, le propanol et l'isopropanol, les alcanediols tels que l'éthylèneglycol, le propylène glycol et le pentanediol, l'alcool benzylique, les éthers en C₂-C₆, les esters en C₂-C₆, la N-méthylpyrrolidone (NMP), et les cétones en C₃-C₆.

20. Composition selon l'une quelconque des revendications précédentes **caractérisée en ce qu'**elle se présente sous forme aqueuse, de préférence sous la forme d'une lotion, épaissie ou non, d'une crème, épaissie ou non, ou d'un gel.

21. Procédé de déformation permanente des cheveux comprenant :
- une étape d'application sur les cheveux d'une composition réductrice telle que définie dans l'une quelconque des revendications 1 à 20, pour réduire les liaisons disulfures de la kératine, puis
- une étape de fixation par oxydation, pour reformer lesdites liaisons, par application d'une composition oxydante, pour reformer lesdites liaisons, par application d'une composition oxydante sur les cheveux ou par mise en contact des cheveux avec l'oxygène de l'air.

22. Procédé selon la revendication 21 **caractérisé en ce que** le ou les photooxydants sont incorporés dans la composition réductrice au moment de l'emploi de la composition.

23. Procédé de déformation permanente des cheveux comprenant :
- une étape d'application sur les cheveux d'une composition cosmétique comprenant, dans un milieu cosmétiquement acceptable, au moins un photooxydant complexé avec au moins un métal tel que défini précédemment, puis
- une étape d'application sur les cheveux d'une composition réductrice comprenant, dans un milieu cosmétiquement acceptable, au moins un agent réducteur,
- une étape de fixation par oxydation, pour reformer lesdites liaisons, par application d'une composition oxydante sur les cheveux ou par mise en contact des cheveux avec l'oxygène de l'air.

24. Procédé selon l'une quelconque des revendications 21 à 23 **caractérisé en ce que** la photooxydation est déclenchée par exposition des cheveux à la lumière naturelle ou artificielle.

25. Procédé selon l'une quelconque des revendications 21 à 24 **caractérisé en ce que** on laisse agir la composition réductrice pendant 5 à 60 minutes, de préférence pendant 15 à 45 minutes.

26. Procédé selon l'une quelconque des revendications 21 à 25 **caractérisé en ce que** la composition oxydante comprend au moins un agent oxydant choisi parmi l'eau oxygénée, les bromates alcalins, les persels, les polythionates et un mélange de bromate alcalin et de persel.

27. Procédé selon l'une quelconque des revendications 21 à 26 **caractérisé en ce qu'**il comprend, entre l'étape d'application sur les cheveux de la composition réductrice et l'étape de fixation, une étape de chauffage des cheveux avec un fer chauffant à une température comprise entre 60 et 250°C, de préférence entre 120 et 220°C.

28. Kit pour la déformation permanente des cheveux comprenant dans un premier compartiment une composition réductrice telle que définie dans l'une quelconque des revendications 1 à 20, et dans un second compartiment une composition oxydante.

## Claims

1. A cosmetic reducing composition to permanently reshape the keratinous fibers, in particular of the hair, **characterized in that** it comprises, in a cosmetically acceptable medium, at least one reducing agent and at least one photo-oxidizing agent complexed with at least one transition metal selected from the group consisting of Ti, V, Cr, Mn, Fe, Co, Ni, Cu, Zn, Mo, Ru, Rh, Pd and Ag, the photo-oxidizing agent (s) is/are selected from :
- porphyrine derivatives selected from chlorines, pheophorbides, pyreophorbides and benzoporphyrines,
- naphtalocyanines,
- phtalocyanines; and
- cyanocobalamine
The photo-oxidizing agent (s) representing 1 to 10% by weight based on the total weight of the composition.

2. A composition according to claim 1, **characterized in that** the transition metal is selected form Cu, Zn, Ni, Fe, Co and Mn.

3. A composition according to anyone of the preceding claims, **characterized in that** the metal is provided as a metal salt, a metal oxide or a metal complex with one or more ligand(s).

4. A composition according to anyone of the preceding claims, **characterized in that** the metal is provided as a metal salt having the following formula:
M(T)ₙ (I)
wherein
M is a metal selected from the group consisting of Ti, V, Cr, Mn, Fe, Co, Ni, Cu, Zn, Mo, Ru, Rh, Pd and Ag,
T is an inorganic anion selected from the group consisting of sulfate, sulfonate, phosphate, phosphonate, chloride, bromide and nitrate ions, or an organic anion selected from the group consisting of lactate, citrate and acetate ions; and
n is an integer ranging from 1 to 8, preferably n is 2 or 3.

5. A composition according to claim 3, **characterized in that** the metal is provided as a metal oxide having the following formula:
M_{m'}(O)ₘ (II)
wherein
M is a metal selected from the group consisting of Ti, V, Cr, Mn, Fe, Co, Ni, Cu, Zn, Mo, Ru, Rh, Pd and Ag,
m' is an integer ranging from 1 to 5 and m is an integer ranging from 1 to 8, preferably m is 2 or 3.

6. A composition according to claim 3, **characterized in that** the metal is provided as a metal complex with one or more ligand(s) having the following formula:
M(L)ₚ (III)
wherein
M is a metal selected from the group consisting of Ti, V, Cr, Mn, Fe, Co, Ni, Cu, Zn, Mo, Ru, Rh, Pd and Ag,
L is an organic ligand selected from saturated or unsaturated, cyclic or acyclic compounds, comprising carboxylate and/or primary, secondary or tertiary amine and/or hydroxy groups, or an inorganic ligand selected from NH₃ and NO; and
p is an integer ranging from 1 to 10, preferably p is 2 or 3.

7. A composition according to claim 6, **characterized in that** the ligand(s) is/are selected from the group consisting of 1,4,8,11-tetraazacyclotetradecane, 1,8-diazabicyclo[5.4.0]undeo-7-ene, ethylenediamine, nitrilotriacetic acid, tris(aminoethyl)amine, tris(aminomethyl)methane, 1,3,5-triaminocyclohexane, dicarboxylic 2,6-pyridine, 2,6-pyridine-dicarboxylic acid, 2,2',6',2"-terpyridine, 1,10-phenanthroline, tris(2-pyridylmethyl)amine, tris(2-pyridylethyl)amine and N-carboxymethyl-N-(2-pyridylmethyl)glycine.

8. A cosmetic composition according to anyone of the preceding claims, **characterized in that** the phtalocyanine(s) complexed with at least one metal is/are selected from compounds of formula: 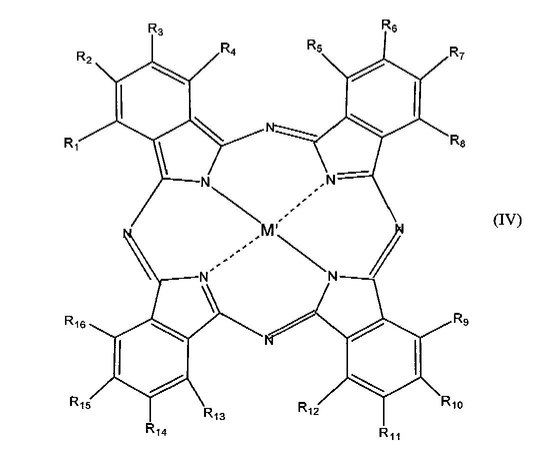 wherein
M' is a metal selected from the group consisting of Ti, V, Cr, Mn, Fe, Co, Ni, Cu, Zn, Mo, Ru, Rh, Pd and Ag, preferably Cu, Zn, Ni, Fe, Co and Mn, more preferably Fe and Mn, said M' metal having an oxidation number of more than 0 and having optionally one or more organic or inorganic anions such as defined in claim 5 and/or one or more ligand(s) such as defined in claim 7,
R₁ to R₁₆ represent independently:
- either hydrogen,
- either hydroxy, amino, mono or dialkylamino, mono or dihydroxyalkylamino, alkoxy, halogeno, cyano moieties,
- either saturated or unsaturated, linear or branched hydrocarbon chains comprising from 1 to 50 carbon atoms, optionally interrupted by one or more heteroatom(s) such as S, O, N, P, by one or more optionally substituted, aromatic or heteroaromatic rings, especially by hydroxy or alkylhydroxy groups or by one or more carbonyl moieties, said chains being optionally substituted by one or more moieties selected from hydroxy, amino, alkoxy, halogeno, cyano, mono or dialkylamino, mono or dihydroxyalkylamino moieties,
- either radicals having solubilizing ionic groups,
- either radicals having one or more reactive groups that are able to graft phtalocyanine onto a solid carrier or onto a soluble or insoluble polymer.

9. A composition according to claim 8, **characterized in that** the ionic solubilizing groups are selected from carboxylate, sulfonate, trialkylammonium, trialkylphosphonium, triarylphosphonium, alkylpyridinium and arylpyridinium groups, alkyl residues being C₁-C₁₈ and aryl residues C₆-C₁₂.

10. A composition according to anyone of the preceding claims, **characterized in that** the photo-oxidizing agent(s) is/are solubilized or dispersed in the composition.

11. A composition according to anyone of claims 1 to 10, **characterized in that** the photo-oxidizing agent(s) is/are adsorbed or grafted onto a solid carrier.

12. A composition according to claim 11, **characterized in that** the solid carrier is selected from the group consisting of silica particles, titanium oxide particles, polymer particles and metal particles.

13. A composition according to claim 12, **characterized in that** the polymer particles are selected from polypropylene, polyurethane, or styrene-divinylbenzene particles.

14. A composition according to anyone of claims 1 to 10, **characterized in that** the photo-oxidizing agent(s) is/are entrapped within a porous material preferably selected from the group consisting of polymer particles, three-dimensional lattices resulting from alkoxy silane molecules condensation and zeolites.

15. A composition according to anyone of the preceding claims, **characterized in that** the reducing agent(s) is/are selected from reducing agents having the following formula:
(H)_{q}X(R)ᵣ (VI)
wherein X is P, S or SO₂, q is 0 or 1, r is 1 or 2 or 3 and R is a linear or branched, saturated or unsaturated (C₁-C₂₀) hydrocarbon radical, optionally interrupted by one heteroatom and having optionally substituents selected from hydroxy, halogeno, amine or carboxy moieties, a ((C₁-C₃₀) alkoxy)carbonyl moiety, an amido moiety, a ((C₁-C₃₀)alkyl)amino carbonyl moiety, a ((C₁-C₃₀)acyl) amino moiety, a mono or di((C₁-C₃₀) alkoxy)carbonyl moiety, a mono or dihydroxy ((C₁-C₃₀)alkyl)amino moiety, or one salt thereof combined with a base.

16. A composition according to anyone of the preceding claims, **characterized in that** the reducing agent(s) is/are selected from the group consisting of thioglycolic acid, thiolactic acid, glycerol monothioglycolate, cysteamine, N-acetyl-cysteamine, N-propionyl-cysteamine, cysteine, N-acetyl-cysteine, thiomalic acid, pantheteine, 2,3-dimercaptosuccinic acid, N-(mercaptoalkyl)-ω-hydroxyalkylamides, N-mono or N,N-dialkylmercapto-4-butyramides, aminomercapto-alkylamides, N-(mercaptoalkyl)succinamic acid derivatives and N-(mercaptoalkyl)succinimide derivatives, alkylamino mercaptoalkylamides, an azeotropic mixture of 2-hydroxypropyl thioglycolate and (2-hydroxy-1-methyl)ethyl thioglycolate, mercaptoalkylaminoamides, N-mercapto-alkylalkanediamides and formamidine sulfinic acid derivatives and the salts thereof.

17. A composition according to anyone of the preceding claims, **characterized in that** the reducing agent(s) represent(s) from 0.05 to 30%, preferably from 1 to 20% by weight, based on the total weight of the composition.

18. A composition according to anyone of the preceding claims, **characterized in that** it comprises at least one surfactant selected from the group consisting of alkyl sulfates, alkyl benzene sulfates, alkyl ether sulfates, alkyl sulfonates, quaternary ammonium salts, alkyl betaines, oxyethylened alkyl phenols, fatty acid alkanolamides, oxyethylened fatty acid esters, and hydroxypropylethers.

19. A composition according to anyone of the preceding claims, **characterized in that** it comprises one or more solvent(s) selected from the group consisting of water, C₁-C₆ alcohols, preferably alkanols such as ethanol, propanol and isopropanol, alkanediols such as ethylene glycol, propylene glycol and pentanediol, benzyl alcohol, C₂-C₆ ethers, C₂-C₆ esters, N-methylpyrrolidone (NMP) and C₃-C₆ ketones.

20. A composition according to anyone of the preceding claims, **characterized in that** it is in aqueous form, preferably as a lotion thickened or not, as a cream thickened or not, or as a gel.

21. A method for permanently reshaping the hair comprising:
- an application step onto the hair of a reducing composition such as defined in anyone of claims 1 to 20, to reduce the keratin disulfide bonds, then
- an oxidizing-fixing step, to form said bonds again, by applying to the hair an oxidizing composition, to form said bonds again, or by contacting the hair with atmospheric oxygen.

22. A method according to claim 21, **characterized in that** the photo-oxidizing agent(s) is/are incorporated into the reducing composition when said composition is ready for use.

23. A method for permanently reshaping the hair comprising:
- an application step onto the hair of a cosmetic composition comprising, in a cosmetically acceptable medium, at least one photo-oxidizing agent complexed with at least one metal as defined above, then
- an application step onto the hair of a reducing composition comprising, in a cosmetically acceptable medium, at least one reducing agent,
- an oxidizing-fixing step, to form said bonds again, by applying to the hair an oxidizing composition or by contacting the hair with atmospheric oxygen.

24. A method according to anyone of claims 21 to 23, **characterized in that** the photooxidation reaction is triggered by exposing hair to a natural or artificial light source.

25. A method according to anyone of claims 21 to 24, **characterized in that** the reducing composition is left on for 5 to 60 minutes, preferably for 15 to 45 minutes.

26. A method according to anyone of claims 21 to 25, **characterized in that** the oxidizing composition comprises at least one oxidizing agent selected from the group consisting of hydrogen peroxide, alkaline bromates, persalts, polythionates and a mixture made of alkaline bromate and persalt.

27. A method according to anyone of claims 21 to 26, **characterized in that** it comprises, between the application step onto the hair of the reducing composition and the fixing step, a hair heating step with a heating iron whose temperature ranges from 60 to 250°C, preferably from 120 to 220°C.

28. A kit to permanently reshape the hair, comprising, in a first compartment, a reducing composition such as defined in anyone of claims 1 to 20 and, in a second compartment, an oxidizing composition.

## Patentansprüche

1. Reduzierende kosmetische Zusammensetzung zur dauerhaften Verformung von Keratinfasern, insbesondere von Haaren, **dadurch gekennzeichnet, dass** sie in einem kosmetisch verträglichen Medium wenigstens ein Reduktionsmittel und wenigstens ein Photooxidans, komplexiert mit wenigstens einem Übergangsmetall, ausgewählt aus Ti, V, Cr, Mn, Fe, Co, Ni, Cu, Zn, Mo, Ru, Rh, Pd und Ag, umfasst, wobei das Photooxidans oder die Photooxidantien ausgewählt ist/sind aus:
- Derivaten von Porphyrinen, ausgewählt aus Chlorinen, Pheophorbiden, Pyreophorbiden und Benzoporphyrinen,
- Naphthalocyaninen,
- Phthalocyaninen und
- Cyanocobalamin,
wobei das Photooxidans oder die Photooxidantien 1-10 Gew.-%, bezogen auf das Gesamtgewicht der Zusammensetzung, darstellt/darstellen.

2. zusammensetzung nach Anspruch 1, **dadurch gekennzeichnet, dass** das Übergangsmetall aus Cu, Zn, Ni, Fe, Co und Mn ausgewählt ist.

3. Zusammensetzung nach einem der vorangehenden Ansprüche, **dadurch gekennzeichnet, dass** das Metall in Form eines Metallsalzes, eines Metalloxids oder eines Metallkomplexes mit einem oder mehreren Liganden zugeführt ist.

4. Zusammensetzung nach dem vorangehenden Anspruch, **dadurch gekennzeichnet, dass** das Metall in Form eines Metallsalzes der folgenden Formel zugeführt ist:
M(T)ₙ (I)
worin
M ein Metall, ausgewählt aus Ti, V, Cr, Mn, Fe, Co, Ni, Cu, Zn, Mo, Ru, Rh, Pd und Ag, ist,
T ein anorganisches Anion, ausgewählt aus den Sulfat-, Sulfonat-, Phosphat-, Phosphonat-, Chlorid-, Bromid- und Nitrat-Ionen, oder ein organisches Anion, ausgewählt aus den Lactat-, Citrat-, Acetat-Ionen, ist und
n eine ganze Zahl ist, die von 1 bis 8 variiert, vorzugsweise gleich 2 oder 3 ist.

5. Zusammensetzung nach Anspruch 3, **dadurch gekennzeichnet, dass** das Metall in der Form eines Metalloxids der folgenden Formel zugeführt ist:
M_{m'} (O)ₘ (II)
worin
M ein Metall ist, ausgewählt aus Ti, V, Cr, Mn, Fe, Co, Ni, Cu, Zn, Mo, Ru, Rh, Pd und Ag,
m' eine ganze Zahl ist, die von 1 bis 5 variiert und m eine ganze Zahl ist, die von 1 bis 8 variiert, vorzugsweise gleich 2 oder 3 ist.

6. Zusammensetzung nach Anspruch 3, **dadurch gekennzeichnet, dass** das Metall in der Form eines Metallkomplexes mit einem Liganden oder mehreren Liganden der folgenden Formel zugeführt ist:
M(L)ₚ (III)
worin
M ein Metall ist, ausgewählt aus Ti, V, Cr, Mn, Fe, Co, Ni, Cu, Zn, Mo, Ru, Rh, Pd und Ag,
L ein organischer Ligand, ausgewählt aus cyclischen oder acyclischen, gesättigten oder ungesättigten Verbindungen, die Carboxylat- und/oder primäre, sekundäre oder tertiäre Amin- und/oder Hydroxygruppen umfassen, oder ein anorganischer Ligand, ausgewählt aus NH₃ und NO, ist und
p eine ganze Zahl ist, die von 1 bis 10 variiert, vorzugsweise gleich 2 oder 3 ist.

7. Zusammensetzung nach Anspruch 6, **dadurch gekennzeichnet, dass** der Ligand oder die Liganden ausgewählt ist/sind aus 1,4,8,11-Tetraazacyclotetradecan, 1,8-Diazabicyclo[5.4.0]undec-7-en, Ethylendiamin, Nitrilotriessigsäure, Tris(aminoethyl)amin, Tris(aminomethyl)methan, 1,3,5-Triaminocyclohexan, Pyridin-2,6-dicarboxyl, Pyridin-2,6-dicarbonsäure, 2,2',6',2"-Terpyridin, 1,10-Phenanthrolin, Tris(2-pyridylmethyl)amin, Tris(2-pyridylethyl)amin, N-Carboxymethyl-N-(2-pyridylmethyl)glycin.

8. Kosmetische Zusammensetzung nach einem der vorangehenden Ansprüche, **dadurch gekennzeichnet, dass** das Phthalocyanin oder die Phthalocyanine, komplexiert mit wenigstens einem Metall, ausgewählt sind aus den Verbindungen der Formel: 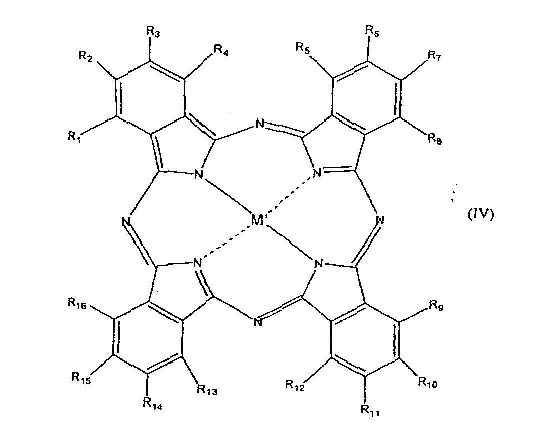 worin
M' ein Metall ist, ausgewählt aus Ti, V, Cr, Mn, Fe, Co, Ni, Cu, Zn, Mo, Ru, Rh, Pd und Ag, vorzugsweise aus Cu, Zn, Ni, Fe, Co und Mn, am Besten Fe und Mn, wobei das Metall M' eine Oxidationsstufe über 0 hat und gegebenenfalls mit einem oder mehreren organischen oder anorganischen Anionen, wie in Anspruch 5 definiert, und/oder einem oder mehreren Liganden, wie in Anspruch 7 definiert, verbunden ist,
R₁ bis R₁₆ unabhängig voneinander darstellen:
- entweder Wasserstoff,
- oder Hydroxy-, Amino-, Mono- oder Dialkylamino-, Mono- oder Dihydroxyalkylamino-, Alkoxy-, Halogen-, Cyano-Gruppierungen,
- oder gesättigte oder ungesättigte, lineare oder verzweigte Kohlenwasserstoffketten, die 1 bis 50 Kohlenstoffatome umfassen, die gegebenenfalls durch ein oder mehrere Heteroatome wie S, O, N, P, durch einen oder mehrere aromatische oder heteroaromatische Cyclen, die gegebenenfalls substituiert sind, insbesondere mit Hydroxy- oder Alkylhydroxygruppen, oder durch ein oder mehrere Carbonyl-Gruppierungen unterbrochen sind, wobei die Ketten gegebenenfalls mit einer oder mehreren Gruppierungen, ausgewählt aus den Hydroxy-, Amino-, Alkoxy-, Halogen-, Cyano-, Mono- oder Dialkylamino-, Mono- oder Dihydroxyalkylamino-Gruppierungen, substituiert sind,
- oder Reste, die solubilisierende ionische Gruppen tragen,
- oder Reste, die eine oder mehrere reaktive Gruppen tragen, die geeignet sind, das Phthalocyanin auf einen festen Träger oder auf ein lösliches oder unlösliches Polymer zu pfropfen.

9. Zusammensetzung nach Anspruch 8, **dadurch gekennzeichnet, dass** die solubilisierenden ionischen Gruppen ausgewählt sind aus den Carboxylat-, Sulfonat-, Trialkylammonium-, Trialkylphosphonium-, Triarylphosphonium-, Alkylpyridinium- und Arylpyridinium-Gruppen, wobei die Alkylreste C₁-C₁₈ und die Arylreste C₆-C₁₂ sind.

10. Zusammensetzung nach einem der vorangehenden Ansprüche, **dadurch gekennzeichnet, dass** das Photooxidans oder die Photooxidantien in der Zusammensetzung solubilisiert ist/sind oder als Dispersion in der Zusammensetzung ist/sind.

11. Zusammensetzung nach einem der Ansprüche 1 bis 10, **dadurch gekennzeichnet , dass** das Photooxidans oder die Photooxidantien auf einem festen Träger adsorbiert ist/sind oder gepfropft ist/sind.

12. Zusammensetzung nach Anspruch 11, **dadurch gekennzeichnet, dass** der feste Träger ausgewählt ist aus Siliciumdioxid-, Titanoxid-, Polymer- und Metallpartikeln.

13. Zusammensetzung nach Anspruch 12, **dadurch gekennzeichnet, dass** die Polymerpartikel ausgewählt sind aus Polypropylen-, Polyurethan- oder Styrol-Divinylbenzol-Partikeln.

14. Zusammensetzung nach einem der Ansprüche 1 bis 10, **dadurch gekennzeichnet, dass** das Photooxidans oder die Photooxidantien in einem porösen Material eingeschlossen ist/sind, das vorzugsweise unter Polymerpartikeln, dreidimensionalen Harzen, erhalten durch Kondensation von Alkoxysilanmolekülen, und Zeolithen ausgewählt ist.

15. Zusammensetzung nach einem der vorangehenden Ansprüche, **dadurch gekennzeichnet , dass** das Reduktionsmittel oder die Reduktionsmittel ausgewählt ist/sind aus den Reduktionsmitteln der Formel
(H)_{q} X (R)ᵣ (VI)
worin X P, S oder SO₂ darstellt, q den Wert 0 oder 1 hat, r den Wert 1 oder 2 oder 3 hat und R ein linearer oder verzweigter, gesättigter oder ungesättigter (C₁-C₂₀) Kohlenwasserstoffrest, der gegebenenfalls durch ein Heteroatom unterbrochen ist und gegebenenfalls Substituenten umfasst, ausgewählt aus einer Hydroxygruppierung, einer halogenierten Gruppierung, einer Amingruppierung und einer Carboxygruppierung, eine ((C₁-C₃₀)Alkoxy)carbonyl-Gruppierung, eine Amidgruppierung, eine ((C₁-C₃₀)Alkyl)aminocarbonyl-Gruppierung, eine ((C₁-C₃₀)Acyl)amino-Gruppierung, eine Mono- oder Di((C₁-C₃₀)alkoxy)carbonyl-Gruppierung, eine Mono- oder Dihydroxy((C₁-C₃₀)alkyl)amino-Gruppierung ist,
oder einem seiner Salze als Kombination mit einer Base.

16. Zusammensetzung nach einem der vorangehenden Ansprüche, **dadurch gekennzeichnet, dass** das Reduktionsmittel oder die Reduktionsmittel ausgewählt ist/sind aus Thioglycolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, N-(Mercaptoalkyl)-ω-hydroxyalkylamiden, N-Mono- oder N,N-Dialkylmercapto-4-butyramiden, Aminomercaptoalkylamiden, Derivaten der N-(Mercaptoalkyl)succinimidsäuren und der N-(Mercaptoalkyl)succinimide, Alkylaminomercaptoalkylamiden, dem azeotropen Gemisch von 2-Hydroxypropylthioglyconat und (2-Hydroxy-1-methyl)ethylthioglycolat, Mercaptoalkylaminoamiden, N-Mercaptoalkylalkandiamiden und Derivaten von Formamidinsulfinsäure und ihren Salzen.

17. Zusammensetzung nach einem der vorangehenden Ansprüche, **dadurch gekennzeichnet , dass** das Reduktionsmittel oder die Reduktionsmittel 0,05 bis 30 Gew.-%, vorzugsweise 1 bis 20 Gew.-%, bezogen auf das Gesamtgewicht der Zusammensetzung, darstellen.

18. zusammensetzung nach einem der vorangehenden Ansprüche, **dadurch gekennzeichnet , dass** sie wenigstens ein grenzflächenaktives Mittel, ausgewählt aus Alkylsulfaten, Alkylbenzolsulfaten, Alkylethersulfaten, Alkylsulfonaten, quaternären Ammoniumsalzen, Alkylbetainen, oxyethylenierten Alkylphenolen, Fettsäurealkanolamiden, oxyethylenierten Fettsäureestern, Hydroxypropylethern, umfasst.

19. Zusammensetzung nach einem der voranstehenden Ansprüche, **dadurch gekennzeichnet, dass** sie ein Lösungsmittel oder mehrere Lösungsmittel, ausgewählt aus Wasser, C₁-C₆-Alkoholen, vorzugsweise Alkanolen wie Ethanol, Propanol und Isopronanol, Alkandiolen wie Ethylenglykol, Propylenglykol und Pentandiol, Benzylalkohol, C₂-C₆-Ethern, C₂-C₆-Estern, N-Methylpyrrolidon (NMP) und C₃-C₆-Ketonen, umfasst.

20. Zusammensetzung nach einem der vorangehenden Ansprüche, **dadurch gekennzeichnet, dass** sie sich in wässriger Form präsentiert, vorzugsweise in der Form einer verdickten oder nicht verdickten Lotion, einer verdickten oder nicht verdickten Creme oder eines Gels.

21. Verfahren zur dauerhaften Verformung der Haare, umfassend:
- eine Stufe der Anwendung bzw. Auftragung einer reduzierenden Zusammensetzung, wie sie in einem der Ansprüche 1 bis 20 definiert ist, auf die Haare, um die Disulfidbindungen des Keratins zu reduzieren, dann
- eine Stufe der Fixierung durch Oxidation, um die genannten Bindungen wieder herzustellen, durch Anwendung bzw. Auftragung einer oxidierenden Zusammensetzung, um die genannten Bindungen wieder herzustellen, durch Anwendung einer oxidierenden Zusammensetzung auf die Haare oder durch In-Kontakt-Bringen der Haare mit dem Sauerstoff der Luft.

22. Verfahren nach Anspruch 21, **dadurch gekennzeichnet, dass** das Photooxidans oder die Photooxidantien zur Zeit der Verwendung der Zusammensetzung in die reduzierende Zusammensetzung eingearbeitet wird/werden.

23. Verfahren zur dauerhaften Verformung der Haare, umfassend:
- eine Stufe der Anwendung bzw. Auftragung einer kosmetischen Zusammensetzung, die in einem kosmetisch verträglichen Medium wenigstens ein Photooxidans, komplexiert mit wenigstens einem Metall, wie vorstehend definiert umfasst, auf die Haare, dann
- eine Stufe der Anwendung bzw. Auftragung einer reduzierenden Zusammensetzung, die in einem kosmetisch verträglichen Medium wenigstens ein Reduktionsmittel umfasst, auf die Haare,
- eine Stufe der Fixierung durch Oxidation, um die genannten Bindungen wieder herzustellen, durch Anwendung bzw. Auftragung einer oxidierenden Zusammensetzung auf die Haare oder durch In-Kontakt-Bringen der Haare mit dem Sauerstoff der Luft.

24. Verfahren nach einem der Ansprüche 21 bis 23, **dadurch gekennzeichnet, dass** die Photooxidation durch Exposition der Haare gegenüber natürlichem oder künstlichem Licht ausgelöst wird.

25. Verfahren nach einem der Ansprüche 21 bis 24, **dadurch gekennzeichnet, dass** man die reduzierende Zusammensetzung während 5 bis 60 Minuten, vorzugsweise während 15 bis 45 Minuten, wirken lässt.

26. Verfahren nach einem der Ansprüche 21 bis 25, **dadurch gekennzeichnet, dass** die oxidierende Zusammensetzung wenigstens ein Oxidationsmittel, ausgewählt aus oxyigeniertem Wasser, Alkalibromaten, Persalzen, Polythionaten und einem Gemisch aus Alkalibromat und Persalz, umfasst.

27. Verfahren nach einem der Ansprüche 21 bis 26, **dadurch gekennzeichnet, dass** es zwischen der Stufe der Anwendung bzw. Auftragung der reduzierenden Zusammensetzung auf die Haare und der Stufe der Fixierung eine Stufe der Erwärmung der Haare mit einem Lockenstab (fer chauffant) bei einer Temperatur zwischen 60 und 250°C, vorzugsweise zwischen 120 und 220°C, umfasst.

28. Kit für die dauerhafte Verformung der Haare, umfassend in einem ersten Fach eine reduzierende Zusammensetzung, wie sie in einem der Ansprüche 1 bis 20 definiert ist, und in einem zweiten Fach eine oxidierende Zusammensetzung.
